(19) Europäisches Patentamt
European Patent Office
Office européen des brevets

(11) Numéro de publication : 0 194 164 B1

# (12) FASCICULE DE BREVET EUROPEEN

(45) Date de publication du fascicule du brevet : **13.03.91 Bulletin 91/11**

(51) Int. Cl.$^5$ : **C07D 253/06**, A61K 31/53

(21) Numéro de dépôt : **86400027.8**

(22) Date de dépôt : **08.01.86**

## (54) N-carboxyalcoyl-2 oxo-3 diaryl 5-6 triazines utiles en thérapeutique.

Le dossier contient des informations techniques présentées postérieurement au dépôt de la demande et ne figurant pas dans le présent fascicule.

(30) Priorité : **04.02.85 FR 8501613**

(43) Date de publication de la demande : **10.09.86 Bulletin 86/37**

(45) Mention de la délivrance du brevet : **13.03.91 Bulletin 91/11**

(84) Etats contractants désignés : **AT BE CH DE GB IT LI LU NL SE**

(56) Documents cités :
**EP-A- 0 036 357**
**FR-A- 2 500 830**

(73) Titulaire : **PIERRE FABRE MEDICAMENT**
**125, Rue de la Faisanderie**
**F-75116 Paris (FR)**

(72) Inventeur : **Pitet, Guy**
**Faculté de Pharmacie 31 allées Jules Guesde**
**F-31400 Toulouse (FR)**
Inventeur : **Cousse, Henry**
**La Foun de los Nobios Chemin de Lastinos**
**F-81100 Castres (FR)**
Inventeur : **Mouzin, Gilbert**
**21, rue Sainte-Foy**
**F-81100 Castres (FR)**

(74) Mandataire : **Doat, Jean-Pierre**
**PIERRE FABRE S.A. Service Propriété Industrielle 17, Avenue Jean Moulin**
**F-81106 Castres Cédex (FR)**

EP 0 194 164 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention, réalisée au Centre de Recherches P.F. MEDICAMENT a pour objet de nouveaux dérivés N-Carboxyalcoyl-2 oxo-3 diaryl 5-6 as triazines, leur procédé de synthèse et leur application thérapeutique. Les nouveaux composés chimiques répondent à la formule générale I :

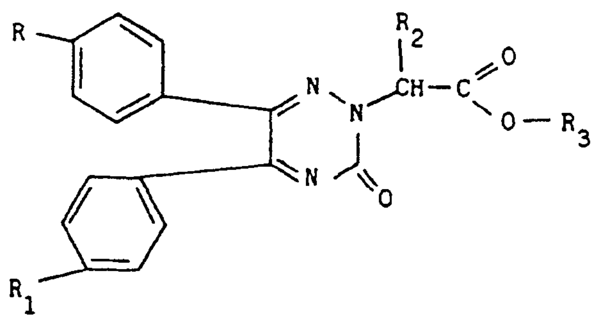

dans laquelle

R et $R_1$ peuvent être identiques ou différents et représentent un hydrogène, un groupe alcoyle, hydroxy, alcoxy, dialcoylamino, ou un halogène. $R_2$ représente un hydrogène ou un groupe alcoyle.
et $R_3$ représente un groupement alcoyle inférieur linéaire ou ramifié.

Des travaux antérieurs, effectués sur des dérivés triaziniques comportant sur l'atome d'azote en position 2 divers groupes fonctionnels, sont illustrés par les brevets n° 77 07245, n° 80 05859 et n° 81 04260 déposés au nom de la demanderesse.

Plus récemment, le brevet n° 83 06107, relatif aux N-cyclo-alcoylméthyl-2 oxo-3 5-6 as triazines, a montré l'importance de certains substituants particuliers. La poursuite des investigations sur cette structure as triazinique a conduit aux nouveaux dérivés, objet de la présente invention, de formule I, dans lesquels le groupe fonctionnel en position-2 est un alcoyl carboxyalcoyle.

De manière tout-à-fait inattendue, ces dérivés ne possèdent que peu ou pas de propriétés anti-inflammatoires. Par contre, leur activité antalgique est très puissante. Certains composés présentent un effet sur le test des contorsions à la phényl benzoquinone :

Exemple 6 : $ED_{50}$ = 13 mg/kg (Aspirine = 100 mg/kg)
(Glafénine = 35 mg/kg). Exemples 9, 10 et 11 = activité 100 mg/kg ≥ - 80%).

A titre d'exemples non limitatifs, il est décrit ci-après quelques composés chimiques et leur mode d'obtention.

<u>Schéma de synthèse</u> :

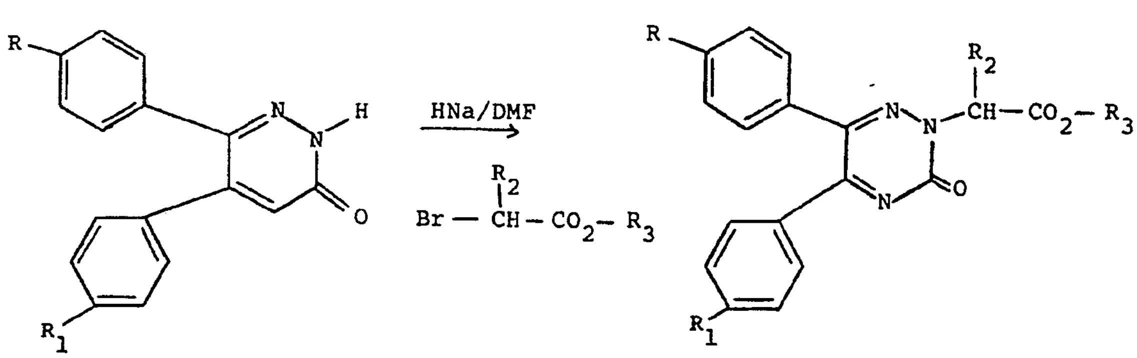


Les intermédiaires triaziniques non substitués en 2 ont été préparés antérieurement par la demanderesse à partir d'α dicétones

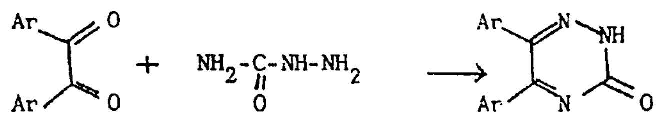

Ar = groupes aryles substitués ou non.

Mode opératoire :

1 mole de triazine en solution dans le diméthyl formamide est traitée par une mole d'hydrure de sodium puis par la quantité stoechiométrique de réactif halogéné.

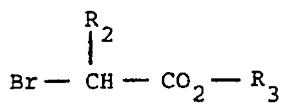

Exemple 1 : Ethoxy carbonyl méthyl-2 oxo-3 di (paraméthoxyphényl) 5,6 as triazine

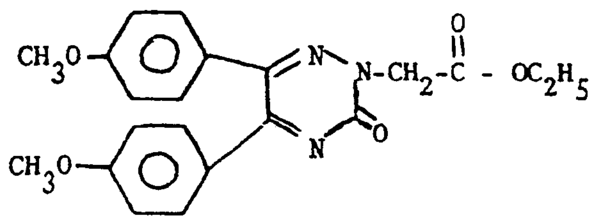

Cristaux jaunes
Soluble dans le chlorure de méthylène, le chloroforme, l'éther ; les alcools et le benzène à chaud.
Insoluble dans l'eau, les acides et les bases dilués.
Point de fusion : 153°C ± 1°C
CCM : benzène – acétate d'éthyle (7/3) – Rf : 0.23
IR : ν C=0 : 1745 $cm^{-1}$.

Exemple 2 : (éthoxycarbonyl-1 éthyl-1)-2 oxo-3 di (parahydroxy phényl) 5,6 as triazine

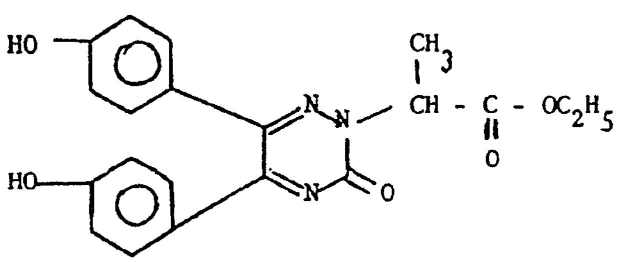

Cristaux jaunes
Soluble dans les alcools à chaud.
Insoluble dans l'eau, acides et bases dilués, le benzène, chloroforme, chlorure de méthylène, éther.
Point de fusion : 276°C
CCM : chloroforme – acétone (7/3) – Rf : 0. 37

IR : ν C0 = 1745 $cm^{-1}$.

Exemple 3 : (éthoxycarbonyl-1 éthyl-1)-2 oxo-3 (paraméthoxyphényl) 5-6 as triazine

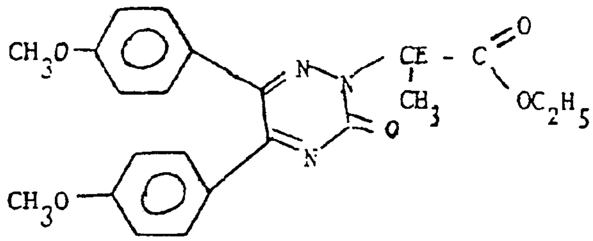

Cristaux jaunes
Soluble dans les alcools à chaud benzène, chloroforme, chlorure de méthylène, éther.
Insoluble dans l'eau, les acides et bases dilués
Point de fusion : 132°C
CCM : éther isopropylique, dioxanne, triéthylamine (85/12.5/2.5)

Exemple 4 : (Ethoxy carbonyl-1 propyl-1)-2 oxo-3 di (paraméthoxy phényl) 5,6 as triazine

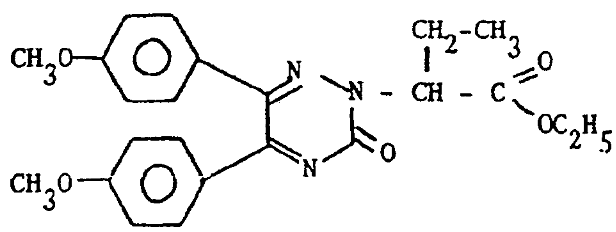

Cristaux jaunes
Soluble dans les alcools à chaud, benzène, chlorure de méthylène, chloroforme.
Insoluble dans l'eau, acides et bases dilués, éther
Point de fusion : 110°C

Exemple 5 : (éthoxy carbonyl-1 butyl-1)-2 oxo-3 di (para méthoxy phényl) 5,6 as triazine

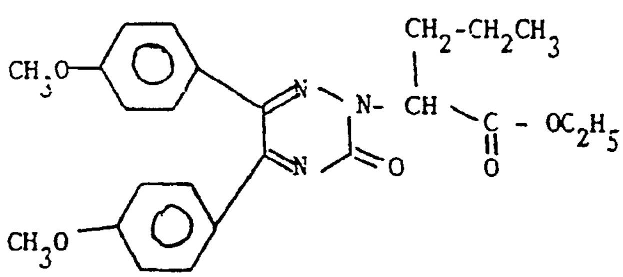

Cristaux jaunes
Soluble dans les alcools à chaud ; benzène, chlorure de méthylène chloroforme, éther
Insoluble dans l'eau, acides et bases dilués
Point de fusion : 110°C

Exemple 6 : (méthoxy carbonyl-1 éthyl)-2 oxo-3 di (paraméthoxy phényl) 5,6 as triazine

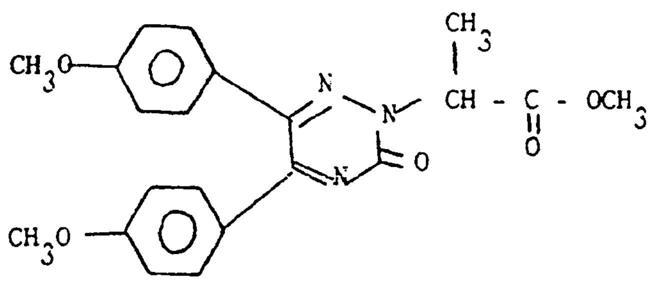

Cristaux jaunes
Soluble dans les alcools à chaud
Insoluble : eau, ether
Point de fusion : 137% C

Exemple 7 : (éthoxy carbonyl-1 éthyl-1)-2 oxo-3 di(phényl) 5,6 as triazine

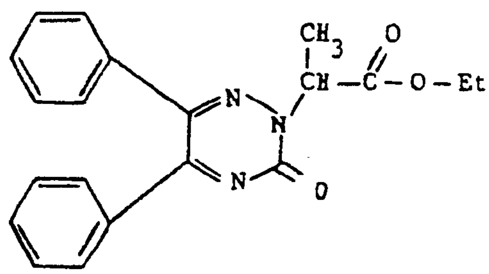

Exemple 8 : (éthoxy carbonyl-1 éthyl-1)-2 oxo-3 di(p-méthylphényl) 5,6 as triazine

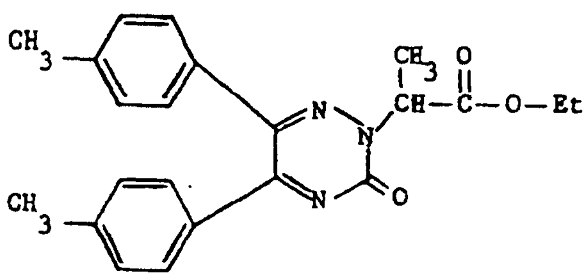

Exemple 9 : (éthoxycarbonyl-1 éthyl-1)-2 oxo-3 di(p-chlorophényl) 5,6 as triazine

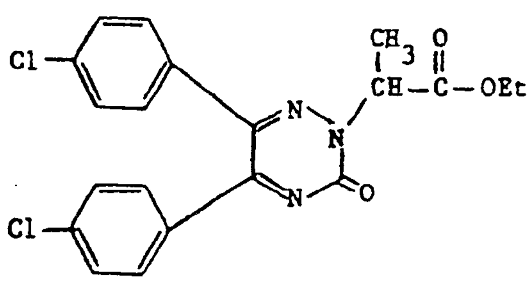

Exemple 10 : (éthoxycarbonyl-1 éthyl-1)-2 oxo-3 (p-chloro p-méthoxy) 5,6 as triazine

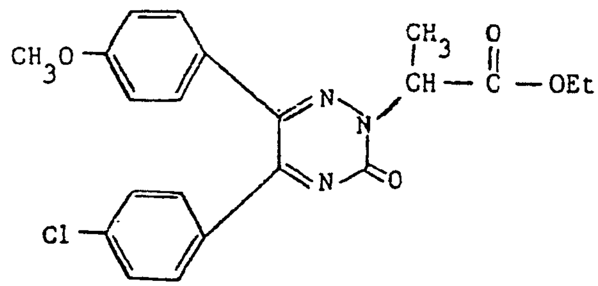

## EXPERIMENTATIONS PHARMACOLOGIQUES

Les expérimentations pharmacologiques ont été effectuées au laboratoire de Pharmacologie du Centre de Recherches P.F MEDICAMENT.

### A) Toxicologie

L'étude de toxicité a été effectuée chez la souris conventionnelle pesant environ 20 grammes.

Les substances ont été administrées par voie orale et les doses létales 50 exprimées en mg/kg et calculées selon la méthode de MILLER et TAINTER – Proc. Soc. Exper. Biol. Med. 1944, 57, 261.

Résultats : Tous les produits testés et décrits dans les exemples ci-dessus ont une $DL_{50}$ supérieure à 1000 mg/kg.

### B) Propriétés antalgiques

L'activité sur le test des contorsions à la phényl benzoquinone (PBQ) selon SIEGMUNG et coll., J. Pharm. Exptl. Ther. 1957, 119, 453, a été déterminée par administration du produit per os.

La dose exprimée en mg/kg chez la souris, 30 mn avant l'injection intrapéritonéale de l'agent algogène. Les résultats sont exprimés en pourcentage de variation du nombre de contorsions.

Pour les molécules les plus actives nous avons déterminé la $DE_{50}$.

| COMPOSES | ACTIVITE $DE_{50}$ mg/kg | 100 mg/kg % inhibition |
|---|---|---|
| Exemple 2 | | - 8 % |
| Exemple 1 | | - 45 % |

| COMPOSES | $DE_{50}$ mg/kg | 100 mg/kg % inhibition |
|---|---|---|
| Exemple 5 | | - 75 % |
| Exemple 4 | | - 83 % |
| Exemple 3 | 13 | |
| Aspirine | 100 | - 50 % |
| Glafénine | 35 | |

## APPLICATIONS THERAPEUTIQUES

Compte tenu de leur parfaite tolérance et de leurs propriétés pharmacologiques, les produits et plus particulièrement ceux décrits aux exemples 3, 4 et 5 peuvent être utilisés dans le traitement des algies d'origines diverses (dentaire, musculaire, rhumatismale).

Les préparations pharmaceutiques contenant les principes actifs peuvent être administrées par voie orale, parentérale ou rectale. Ces compositions pharmaceutiques peuvent également contenir, en association, d'autres principes actifs pharmaceutiquement et thérapeutiquement acceptables.

La dose par unité de prise sera par exemple comprise entre environ 10 et environ 100 mg.

## Revendications

1. A titre de composés chimiques nouveaux, les dérivés de formule générale :

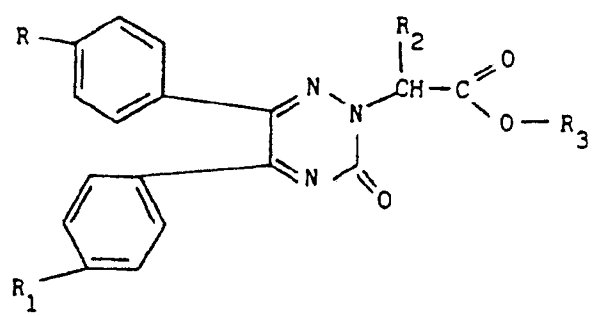

dans laquelle :

R et $R_1$ peuvent être identiques ou différents et représentent un hydrogène, un groupe alcoyle, hydroxy, alcoxy, dialcoylamino, ou un halogène.

$R_2$ représente un hydrogène ou un groupe alcoyle.

et $R_3$ représente un groupement alcoyle inférieur linéaire ou ramifié.

2. Les composés chimiques selon la revendication 1 et plus particulièrement :

- Ethoxy carbonyl méthyl-2 oxo-3 di(paraméthoxyphényl) 5,6 as triazine
- (Ethoxycarbonyl-1 éthyl-1)-2 oxo-3 di(parahydroxyphényl) 5,6 as triazine
- (Ethoxycarbonyl-1 éthyl-1)-2 oxo-3 di(paraméthoxyphényl) 5,6 as triazine
- (Ethoxy carbonyl-1 propyl-1)-2 oxo-3 di(paraméthoxyphényl) 5-6 as triazine.
- (Ethoxy carbonyl-1 butyl-1)-2 oxo-3 di(paraméthoxyphényl) 5-6 as triazine
- (Méthoxycarbonyl-1 éthyl)-2 oxo-3 di(paraméthoxyphényl) 5-6 as triazine
- (Ethoxy-carbonyl-1 éthyl-1)-2 oxo-3 di(phényl) 5-6 as triazine.
- (Ethoxy-carbonyl-1 éthyl-1)-2 oxo-3 di(paraméthylphényl) 5-6 as triazine
- (Ethoxycarbonyl-1 éthyl-1)-2 oxo-3 di(parachlorophényl) 5-6 as triazine

– (Ethoxycarbonyl-1 éthyl-1)-2 oxo-3(parachloro-paraméthoxy) 5-6 as triazine.

3. Le procédé d'obtention des composés selon les revendications 1 et 2 qui consiste à traiter les triazines de formule :

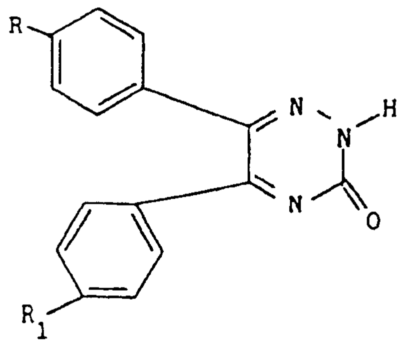

en présence d'un agent sodant par un groupe halogéno alcoylcarboxylate de formule :

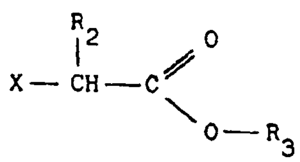

dans laquelle : X représente un halogéne $R_2$ et $R_3$ définis dans la revendication 1.

4. A titre de médicaments nouveaux, utilisables en thérapeutique humaine ou vétérinaire, les composés selon les revendications 1 et 2 doués de propriétés altalgiques.

5. Les compositions pharmaceutiques contenant à titre de principe actif antalgique, un composé selon les revendications 1, 2 et 4 utilisées en thérapeutique.

6. Les compositions selon la revendication 5 contenant d'autres principes actifs venant compléter l'action thérapeutique.

7. Les médicaments selon les revendications 4, 5 et 6 contenant les excipients appropriés et les véhicules galéniques permettant l'administration par voie orale, locale, injectable ou rectale.

## Ansprüche

1. Neue chemische Verbindungen, nämlich Derivate der allgemeinen Formel :

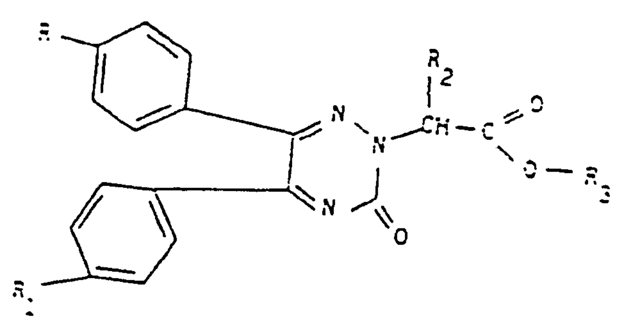

worin

R und $R_1$, gleich oder verschieden sein können und ein Wasserstoffatom, eine Alkylgruppe, Hydroxy, Alkoxy, Dialkylamino, oder ein Halogen bedeuten ; $R_2$ ein Wasserstoffatom oder eine Alkylgruppe bedeutet ; und $R_3$ eine niedere geradkettige oder verzweigte Alkylgruppe bedeutet.

2. Verbindungen nach Anspruch 1, und zwar insbesondere :

– 2-Ethoxycarbonylmethyl-3-oxo-5,6-di(paramethoxyphenyl)-as-triazin

– 2-(1-Ethoxycarbonyl-ethyl)-3-oxo-5,6-di(parahydroxyphenyl)-as-triazin

- 2-(1-Ethoxycarbonyl-ethyl)-3-oxo-5,6-di(paramethoxyphenyl)-as-triazin
- 2-(1-Ethoxycarbonyl-propyl)-3-oxo-5,6-di(paramethoxyphenyl)-as-triazin
- 2-(1-Ethoxycarbonylbutyl)-3-oxo-5,6-di(paramethoxyphenyl)-as-triazin
- 2-(1-Methoxycarbonyl-ethyl)-3-oxo-5,6-di(paramethoxyphenyl)-as-triazin
- 2-(1-Ethoxycarbonyl-ethyl)-3-oxo-5,6-diphenyl-as-triazin
- 2-(1-Ethoxycarbonyl-ethyl)-3-oxo-5,6-di(paramethylphenyl)-as-triazin
- 2-(1-Ethoxycarbonyl-ethyl)-3-oxo-5,6-di(parachlorphenyl)-as-triazin
- 2-(1-Ethoxycarbonyl-ethyl)-3-oxo-5,6(parachlor-paramethoxy)-as-triazin

3. Verfahren zur Herstellung der Verbindungen gemäß den Ansprüchen 1 und 2, dadurch gekennzeichnet, daß man die Triazine der Formel :

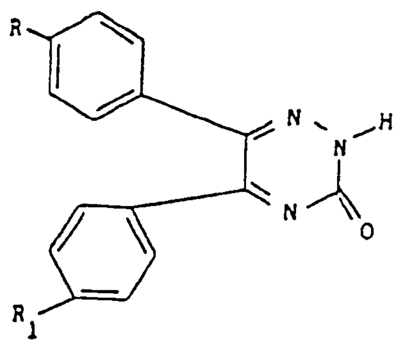

in Gegenwart eines natriumalkalischen Mittels mit einer Halogenalkylcarboxylat-Gruppierung der Formel :

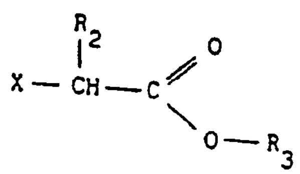

behandelt, worin X ein Halogen bedeutet und $R_2$ und $R_1$ die in Anspruch 1 angegebene Bedeutung besitzen.

4. Verbindungen nach den Ansprüchen 1 und 2 als neue Arzneimittel zur Verwendung in der menschlichen oder veterinärmedizinischen Therapie mit antalgischen (analgetischen) Eigenschaften.

5. Pharmazeutische Zusammensetzungen enthaltend als antalgisches (analgetisches) Wirkprinzip eine Verbindung gemäß einem der Ansprüche 1, 2 und 4 zur therapeutischen Verwendung.

6. Zusammensetzungen nach Anspruch 5, dadurch gekennzeichnet, daß sie auch andere Wirkstoffe zur Vervollständigung der therapeutischen Wirkung enthalten.

7. Arzneimittel nach den Ansprüchen 4, 5 und 6, dadurch gekennzeichnet, daß sie geeignete Exzipientien und galenische Trägerstoffe zur oralen, lokalen, Injektions- oder rektalen Verabreichung enthalten.

## Claims

1. As novel chemical compounds, the derivatives of the general formula :

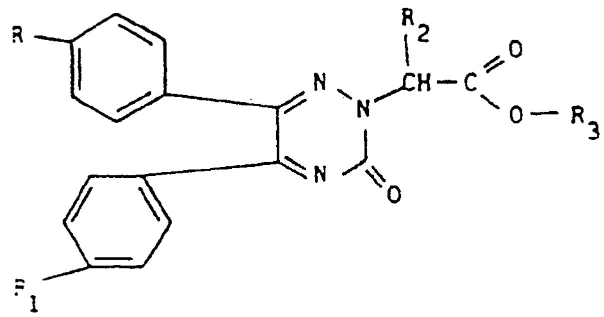

in which :

R and $R_1$ may be identical or different and represent a hydrogen, an alkyl, hydroxy, alkoxy or dialkylamino group, or a halogen,

$R_2$ represents a hydrogen or an alkyl group,

and $R_3$ represents a linear or branched lower alkyl group.

2. The chemical compounds according to claim 1, and more especially :

– 2-ethoxycarbonylmethyl-3-oxo-5,6-di-(paramethoxyphenyl)-as.-triazine,
– 2-(1-ethoxycarbonyl-1-ethyl)-3-oxo-5,6-di-(parahydroxyphenyl)-as.-triazine,
– 2-(1-ethoxycarbonyl-1-ethyl)-3-oxo-5,6-di-(paramethoxyphenyl)-as.-triazine,
– 2-(1-ethoxycarbonyl-1-propyl)-3-oxo-5,6-di-(paramethoxyphenyl)-as.-triazine,
– 2-(1-ethoxycarbonyl-1-butyl)-3-oxo-5,6-di-(paramethoxyphenyl)-as.-triazine,
– 2-(1-methoxycarbonylethyl)-3-oxo-5,6-di-(paramethoxyphenyl)-as.-triazine,
– 2-(1-ethoxycarbonyl-1-ethyl)-3-oxo-5,6-di-(phenyl)-as.-triazine,
– 2-(1-ethoxycarbonyl-1-ethyl)-3-oxo-5,6-di-(paramethylphenyl)-as.-triazine,
– 2-(1-ethoxycarbonyl-1-ethyl)-3-oxo-5,6-di-(parachlorophenyl)-as.-triazine,
– 2-(1-ethoxycarbonyl-1-ethyl)-3-oxo-5,6-(parachloroparamethoxy)-as.-triazine.

3. Process for obtaining compounds according to claims 1 and 2, which comprises treating the triazines of the formula :

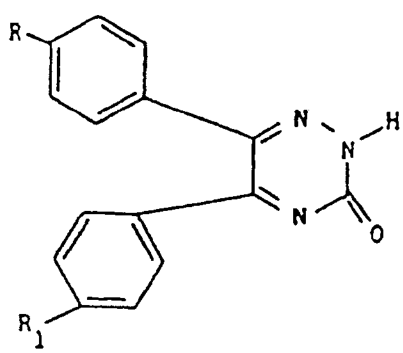

in the presence of a sodium-providing agent with a haloalkylcarboxylate group of the formula :

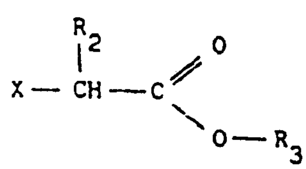

in which : X represents a halogen and $R_2$ and $R_3$ are as defined in claim 1.

4. Compounds according to claims 1 and 2 having antalgesic properties, as novel medicaments that can be used in human or veterinary therapeutics.

5. Pharmaceutical compositions that contain as the antalgesic active ingredient a compound according to claims 1, 2 and 4 and that are used in therapeutics.

6. Compositions according to claim 5 containing other active ingredients that complement the therapeutic action.

7. Medicaments according to claims 4, 5 and 6 containing the appropriate excipients and the galenical vehicles that permit administration orally, locally, by injection or rectally.